# EUROPÄISCHE PATENTSCHRIFT

(11) **EP 1 631 939 B1**
(45) Veröffentlichungstag und Bekanntmachung des Hinweises auf die Patenterteilung: **19.06.2019**
(21) Anmeldenummer: 04739174.3
(22) Anmeldetag: 11.05.2004
(51) Int. Cl.: G07C 11/00, G07C 9/00, A61M 5/145

(54) **VORRICHTUNG UND VERFAHREN ZUR ERKENNUNG EINES BENUTZERS EINES MEDIZINISCHEN GERÄTS**
DEVICE AND METHOD FOR IDENTIFYING A MEDICAL DEVICE USER
DISPOSITIF ET PROCÉDÉ D'IDENTIFICATION D'UN UTILISATEUR D'UN APPAREIL MÉDICAL

(30) Priorität: 03.06.2003 DE 10325106
(43) Veröffentlichungstag der Anmeldung: 08.03.2006
(73) Patentinhaber: F. Hoffmann-La Roche AG, 4070 Basel (CH); Roche Diabetes Care GmbH, 68305 Mannheim (DE)
(72) Erfinder: EBERHART, Andreas, CH-6300 Zug (CH); HEINIGER, Hanspeter, CH-4932 Lotzwil (CH); IMHOF, Erich, CH-3427 Utzenstorf (CH); RUFER, Thomas, CH-3072 Ostermundigen (CH)
(74) Vertreter: Rentsch Partner AG
(86) Internationale Anmeldenummer: PCT/EP2004/005038
(87) Internationale Veröffentlichungsnummer: WO 2004/107277

(56) Entgegenhaltungen:
- EP-A- 0 980 688
- WO-A-01/18332
- WO-A-99/10029
- WO-A-02/099393
- DE-A1- 10 046 406
- US-A- 5 936 523
- US-A1- 2002 034 321
- US-A1- 2002 038 392
- US-A1- 2003 065 308

## Beschreibung

Die vorliegende Erfindung bezieht sich auf eine Vorrichtung zum Erkennen ob ein medizinisches Gerät, wie z.B. ein Injektionsgerät, ein Infusionsgerät oder eine Messvorrichtung z.B. für die Konzentration einer bestimmten Substanz, von einer Person betätigt werden, wobei gemäß einer bevorzugten Aus-führungsform festgestellt werden soll, ob diese Person zur Betätigung des Geräts oder zur Veranlassung einer bestimmten Aktion des Geräts berechtigt ist.

Bei medizinischen Geräten, wie z.B. bei Insulinpumpen., wird häufig gewünscht, dass die Bedienung eines solchen Geräts möglichst unauffällig durchgeführt werden kann,, wobei das Gerät z.B. in der Hosentasche oder unter den Kleidern eines Anwenders mitgeführt wird. Soll z.B. ein Bolus "blind" ausgelöst werden, so sollten die zur Auslösung erforderlichen Tasten oder Schalter einfach fühlbar sein, so dass diese z.B. auch unter den Kleidern erkennbar sind und betätigt werden können. Jedoch ist es bei der Verwendung von einfachen Druckschaltern möglich, dass zufällig und ungewollt durch eine versehentliche Betätigung des Schalters, z.B. durch das Anlegen eines Sicherheitsgurts oder durch einen versehentlichen Stoss an einer Tischkante, ein Bolus ausgelöst wird.

Weiterhin besteht bei medizinischen durch einen einfachen Schalter zu betätigenden Geräten die Gefahr, dass versehentlich oder absichtlich zur Bedienung des Geräts nicht berechtigte Personen, wie z.B. Kinder oder im Umgang mit dem Gerät unerfahrene Personen, unerwünschte Aktionen des Gerätes auslösen.

Die US 2002/0038392 A1 offenbart ein klinisches Infusionssystem. Ein von einem Arzt mitgeführtes Arztidentifikationssystem ist dabei mit einem Transponder ausgestattet, welcher mit einem Transponder von Infusionsgeräten und/oder einem zentralen Controller kommunizieren kann.

Die EP 0 980 688 A2 offenbart eine Inulinpumpe, bei welcher nach Eingabe eines Geheimcodes ein spezieller Arzt-Modus zugänglich ist. Im Arzt-Modus kann eine Obergrenze für zu infundierende Insulinmengen programmiert werden.

Die US 2002/0034321 A1 sowie die DE10046406A1 offenbaren jeweils Fingerprint-Sensoren.

Es ist eine Aufgabe der vorliegenden Erfindung eine Vorrichtung vorzuschlagen, welche die sichere und zuverlässige Bedienung eines medizinischen Geräts ermöglichen.

Die erfindungsgemäße Vorrichtung ist ein medizinisches Gerät mit einem Infusionsgerät in Form einer Insulinpumpe und weist ein Bedienelement zum Auslösen einer Aktion, nämlich der Abgabe einer Substanz durch Injektion oder Infusion, z.B. als Bolus, auf, wobei erfindungsgemäß ein Sicherheitssystem vorgesehen ist, um eine unbeabsichtigte, unberechtigt oder fehlerhafte Auslösung einer Aktion zu verhindern und/oder um mindestens eine Person zu identifizieren.

Allgemein kann ein Sicherheitssystem auf verschiedenen Funktionsprinzipien basieren, welche einzeln oder in Kombination verwendet werden können, um z.B. festzustellen, dass eine Person das medizinische Gerät bzw. die Vorrichtung betätigt hat, d.h., dass das Gerät z.B. nicht fehlerhaft durch einen Sicherheitsgurt oder einen unbeabsichtigten Stoss betätigt wurde. Weiterhin kann das Sicherheitssystem so ausgelegt sein, dass überprüft wird, ob die Person, welche ein Bedienelement betätigt hat, hierzu überhaupt berechtigt ist, so dass - falls erforderlich - die Ausführung der gewünschten Aktion auch gesperrt werden kann, falls der Person die Berechtigung fehlt, so dass z.B. Kinder oder zur Durchführung einer Aktion oder Änderung einer Einstellung wie z.B. einer Veränderung der Dosierungsmenge einer abzugebenden Substanz nicht berechtigte Personen oder Patienten nicht in die von z.B. einem berechtigten Arzt eingestellten Betriebsarten oder Parameter, wie z.B. Dosierungsverteilungen eingreifen können.

Wird eine Person identifiziert, so kann auch eine für diese Person gespeicherte Betriebsart oder z.B. ein Behandlungsplan oder Therapieprogramm in dem Gerät eingestellt werden.

Gemäß eines Beispiels kann das Sicherheitssystem als Kraftsensor ausgebildet sein, wobei die auf ein Bedienelement, wie z.B. einen Schalter wirkende Kraft gemessen wird. Dabei kann z.B. ein Grenzwert einer mindestens aufzubringenden und/oder höchstens anliegenden Kraft vorgesehen sein, so dass eine Aktion erst bei Überschreiten des unteren Grenzwertes und/oder nicht bei Überschreiten des oberen Grenzwertes ausgelöst wird, wodurch eine fehlerhafte Auslösung z.B. durch leichte Stöße vermieden wird. Weiterhin ist es möglich ein zeitliches Profil einer Kraftverteilung vorzugeben, mit welchem das von dem Kraftsensor erfasste tatsächliche Profil der auf das Bedienelement wirkenden Kraft verglichen wird, so dass beispielsweise nur für eine Betätigung durch einen Finger übliche Kraftverteilungen zur Auslösung einer gewünschten Aktion führen und beispielsweise keine "blinde" Bolusauslösung durch versehentliche leichte oder starke Stöße oder durch Kinder, welche gewöhnlich nur eine im Vergleich zu Erwachsenen geringe Kraft aufbringen können, erfolgt.

Ebenso ist es möglich, einen Temperatursensor als Sicherheitssystem zu verwenden, welcher die bei einem Bedienelement vorhandene Temperatur z.B. während der Betätigung des Bedienelements misst, wodurch ermittelt werden kann, ob das Bedienelement z.B. durch eine etwa körperwarme Fingerspitze betätigt wurde.

Des Weiteren kann ein optischer Sensor als Sicherheitssystem verwendet werden, welcher beispielsweise die Oberfläche eines Fingers beispielsweise durch Laserstrahlen abtasten kann, um z.B. zu erkennen, ob ein Bedienelement von einem Finger betätigt wurde, oder um den Fingerabdruck des zur Betätigung des Bedienelements verwendeten Fingers zu erkennen, um anhand des Fingerabdrucks die Berechtigung einer Person zur Auslösung einer bestimmten Aktion zu überprüfen.

Erfindungsgemäss werden sogenannte "Fingerprint"- oder Fingerabdruck-Sensoren als Sicherheitssystem verwendet, welche auf unterschiedlichen Funktionsprinzipien basieren. Solche Fingerprint-Sensoren sind im Stand der Technik bekannt und können beispielsweise auch auf der Messung einer kapazitiven Kopplung zwischen der Oberfläche eines Fingers und dem Sensor basieren, wobei eine Vielzahl relativ kleiner Kapazitäts-Messvorrichtungen auf einer Fläche vorgesehen sind, auf welche eine Fingerspitze gelegt werden soll, um das Profil der Fingerspitze und somit den Fingerabdruck zu erkennen.

Weiterhin kann beispielsweise ein Touch-Screen als Sicherheitssystem verwendet werden, welcher vorteilhaft auch Informationen bezüglich des Betriebszustandes des zu bedienenden medizinischen Geräts und/oder einzustellende Parameter oder Betriebsarten anzeigt.

Weiterhin kann ein Transponder als Bedienelement und/oder Sicherheitssystem verwendet werden, welcher von einer Person getragen und in die Nähe des medizinischen Gerätes gebracht werden muss, von welchem der Transponder erfasst wird, um z.B. das medizinische Gerät für die Bedienung durch diese Person freizuschalten. Es ist auch möglich, dass der Transponder in die Person implantiert wird, so dass eine eindeutige Zuordnung des medizinischen Geräts zu einer bestimmten Person gewährleistet ist.

Allgemein kann das erfindungsgemäße Sicherheitssystem durch eines oder mehrere der oben beschriebenen Systeme gebildet werden, wobei zur Erhöhung der Bediensicherheit zwei oder mehr Sicherheitssysteme bei einem medizinischen Gerät vorgesehen werden können, welche alle auf eine vorgegebene Art aktiviert oder ausgelöst werden müssen, um z.B. eine eindeutige Identifikation eines Benutzers oder die Überprüfung der Berechtigung zur Bedienung. durchzuführen.

Das erfindungsgemäße Sicherheitssystem kann weiterhin auch mit dem Bedienelement derart gekoppelt sein und kann so ausgelegt sein, dass vor dem Auslösen einer Aktion eine Bestätigung durch einen berechtigten Benutzer erforderlich ist, wobei z.B. nach dem Anfordern der Auslösung einer bestimmten Aktion ein optisches und/oder akustisches Signal ausgegeben werden kann, welches einem Benutzer anzeigt, dass die auszulösende Aktion jetzt bestätigt werden muss, bevor diese Aktion, wie z.B. die Abgabe einer Substanz, ausgeführt wird.

Vorteilhaft kann eine Kombination verschiedener Sicherheits- oder Schaltsysteme zum energiesparenden Betreiben eines medizinischen Geräts verwendet werden, wobei beispielsweise ein erster Schalter oder ein erstes Sicherheitssystem vorgesehen ist, welches vor der Betätigung wenig oder keine Energie verbraucht, wie z.B. ein Kippschalter, welcher nach der Betätigung mindestens ein weiteres Sicherheitssystem anschaltet, welches beispielsweise einen erhöhten Energieverbrauch hat, um die Identität einer das medizinische Gerät bedienenden Person zu überprüfen, wobei bei einer fehlgeschlagenen Identifikation und/oder nach Verstreichen einer vorgegebenen Zeit das energieintensivere Sicherheitssystem wieder abgeschaltet wird, um die Energieaufnahme durch das Sicherheitssystem zu minimieren und somit beispielsweise die Lebensdauer einer in dem medizinischen Gerät vorgesehenen Batterie zu erhöhen.

Weiterhin können in einem nicht anspruchsgemässen Beispiel auch zwei einfache Bedienungselemente, wie z.B. zwei an verschiedenen Positionen des medizinischen Geräts angebrachte Schalter, welche gleichzeitig oder in einer vorgegebenen Reihenfolge betätigt werden müssen, verhindern, dass ein z.B. durch einen Stoss versehentlich gedrückter Schalter schon zur unerwünschten Auslösung einer Aktion, wie z.B. der Abgabe eines Bolus, führt. Insbesondere ist es vorteilhaft, die Bedienelemente an zwei Seiten des medizinischen Gerätes anzubringen, welche möglichst nicht gleichzeitig durch einen Stoss eine Krafteinwirkung erfahren.

Vorteilhaft ist das Sicherheitssystem in der Lage mindestens eine Person zu identifizieren. Dies kann z.B. durch die Erfassung eines oder mehrerer biometrischer personenspezifischer Merkmale erfolgen. Bevorzugt kann eine Datenbank z.B. in dem medizinischen Gerät oder separat von diesem Gerät vorgesehen sein, in welche Muster oder allgemeine Informationen bezüglich biometrischer Daten einzelner Personen abgespeichert sind, wobei das medizinische Gerät z.B. auf die Datenbank über eine geeignete Schnittstelle, wie z.B. ein Kabel, Funk oder Infrarotübertragung, zugreifen kann, um Vergleichs- oder Referenzdaten für den Vergleich mit den tatsächlich erfassten biometrischen Daten zur Verfügung zu haben. Hierdurch können z.B. ein oder mehrere Personen eindeutig identifiziert werden und es kann beispielsweise festgestellt werden, ob eine so identifizierte Person zur Durchführung einer gewünschten Aktion berechtigt ist, oder ob die Durchführung der Aktion gesperrt wird. Beispielsweise können biometrische Daten, wie z.B. der Fingerabdruck oder das Netzhaut-Muster, eines zur Einstellung von Parametern des medizinischen Gerätes berechtigten Arztes in einer solchen Datenbank vorgesehen sein, so dass nur diese berechtigte Person z.B. die Dosis einer von dem medizinischen Gerät abzugebenden Substanz einstellen kann. Von anderen, nicht in der Datenbank gespeicherten oder in der Datenbank gespeicherten und mit einer anderen Zugriffsberechtigung versehenen Personen können bestimmte Aktion nicht oder nur zum Teil ausgeführt werden, während es auch möglich ist, dass bestimmte Funktionen von allen Personen ausgeführt werden können, ohne diese Personen zu identifizieren oder deren Berechtigung zu überprüfen. In einer solchen Datenbank können zur Identifikation einer oder mehrerer Personen dienende Daten auch bestimmte Berechtigungsstufen zugeordnet sein.

Bevorzugt können bei einem oder mehreren als Sicherheitssystem vorgesehenen Sensoren Führungen oder Ausrichtungselemente vorgesehen sein, mit welchen bewirkt wird, dass diejenigen Teile einer Person, welche zur Identifikation der Person verwendet werden, auf eine vorgegebene Weise ausgerichtet sind, um vergleichbare Daten bei verschiedenen Messungen zu erhalten. Beispielsweise kann eine Führung für einen Finger vorgesehen sein, so dass eine auf einen Sensor aufzulegende Fingerkuppe immer in etwa gleich ausgerichtet ist, wenn diese auf den Sensor gelegt wird. Hierdurch kann z.B. die Erfassungsgeschwindigkeit des Sensors erhöht werden, da nicht durch zusätzliche Rechenzeit überprüft werden muss, ob etwa ein erfasster Fingerabdruck durch Drehung mit einem z.B. in einer Datenbank gespeicherten Referenz-Fingerabdruck übereinstimmt.

Vorteilhaft kann eine optische und/oder akustische Ausgabevorrichtung, wie z.B. eine LED oder ein Lautsprecher vorgesehen sein, um z.B. den Betriebszustand des medizinischen Geräts anzuzeigen oder um auf einzugebende Befehle aufmerksam zu machen.

Allgemein kann das Sicherheitssystem in oder bei dem medizinischen Gerät vorgesehen sein. Es ist auch möglich, eine separate Vorrichtung vorzusehen, welche mit dem medizinischen Gerät koppelbar ist und zur Erfassung von Identifikationsmerkmalen, wie z.B. biometrischen Daten, dienen kann. Dabei kann die separate Vorrichtung z.B. über Kabel, Funk oder InfrarotSignale mit dem medizinischen Gerät gekoppelt werden, um z.B. Identifikation-Daten oder Daten zur Einstellung des medizinischen Geräts an dieses zu übertragen. Ebenso können auch von dem medizinischen Gerät Daten an die separate Vorrichtung übertragen werden, wie z.B. Daten bezüglich eines aktuellen Betriebszustandes oder aufgezeichnete Daten, welche den Betrieb des medizinischen Geräts über einen vorgegebenen Zeitraum spezifizieren.

Offenbart wird gemäß einem weiteren Aspekt ein Verfahren zum Betätigen eines medizinischen Geräts, wie z.B. zum Auslösen der Abgabe einer Substanz und/oder zum Durchführen einer Messung, wobei erfindungsgemäß ein Überprüfungs- oder Identifikationsverfahren durchgeführt wird, um zu erkennen, ob eine das medizinische Gerät bedienende Person tatsächlich berechtigt ist, den gewünschten Bedienvorgang durchzurühren oder um zu erkennen, ob überhaupt eine Person einen Bedienvorgang ausgelöst hat, oder ob dieser Bedienvorgang z.B. versehentlich nicht durch eine Person, sondern durch einen Stoss ausgelöst wurde, wobei die Ausführung des Vorganges dann nicht begonnen wird.

Die Erfindung wird nachfolgend anhand von Beispielen beschrieben werden. Es zeigen:
- Fig.1: eine schematische Darstellung einer Ausführungsform einer Pumpe;
- Fig. 2: ein Ablaufdiagramm einer ersten Ausführungsform eines Verfahrens; und
- Fig. 3: ein Ablaufdiagramm einer zweiten Ausführungsform eines Verfahrens.

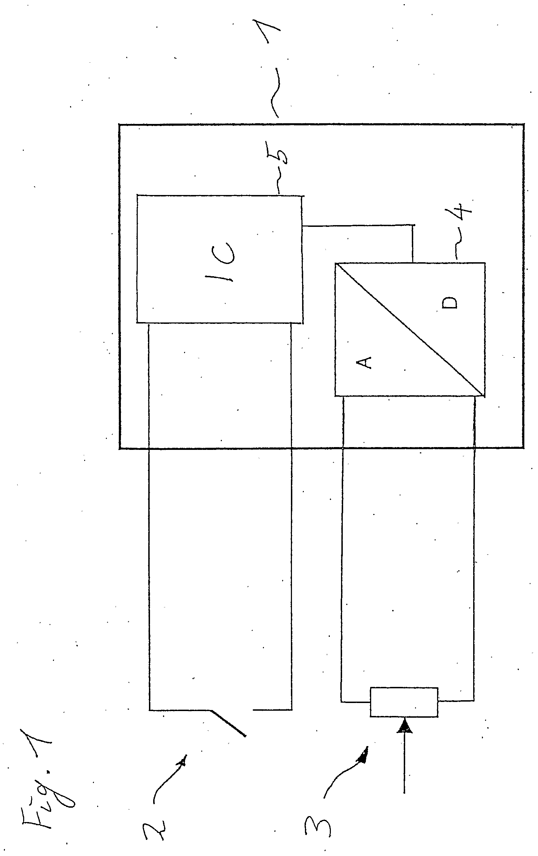

Fig. 1 zeigt schematisch eine Ausführungsform einer Insulinpumpe 1 mit einer Pumpenelektronik 5, wobei zur Bedienung der Pumpe 1 eine Taste 2 und zur Verhinderung einer sogenannten "blinden" Bolusauslösung der Insulinpumpe 1 ein Kraftsensor 3 vorgesehen ist. Die Taste 2 ist so mit dem Kraftsensor 3 gekoppelt, dass ein auf die Taste 2 wirkender Druck bzw. eine Kraft erfasst werden kann und mit einer vorgegebenen Kraft oder einem Druckprofil nach einer Wandlung der analog erfassten Signale in dem A/D-Wandler 4 in der Pumpenelektronik 5 verglichen werden kann. Somit kann ein versehentliches Betätigen der Taste z.B. durch einen Stoss erkannt werden, da in einem solchen Fall die für die Betätigung durch einen Finger typische Kraft nicht am Kraftsensor anliegt bzw. eine fit eine manuelle Tastenbetätigung charakteristische Kraftverteilung oder ein zeitlicher Verlauf der Kraft oder des auf den Kraftsensor 3 wirkenden Druckes nicht mit einem vorgegebenen Wert oder Profil übereinstimmt, welcher z.B. in der Pumpenelektronik 5 gespeichert ist, so dass die fehlerhafte Auslösung eines Bolus verhindert werden kann.

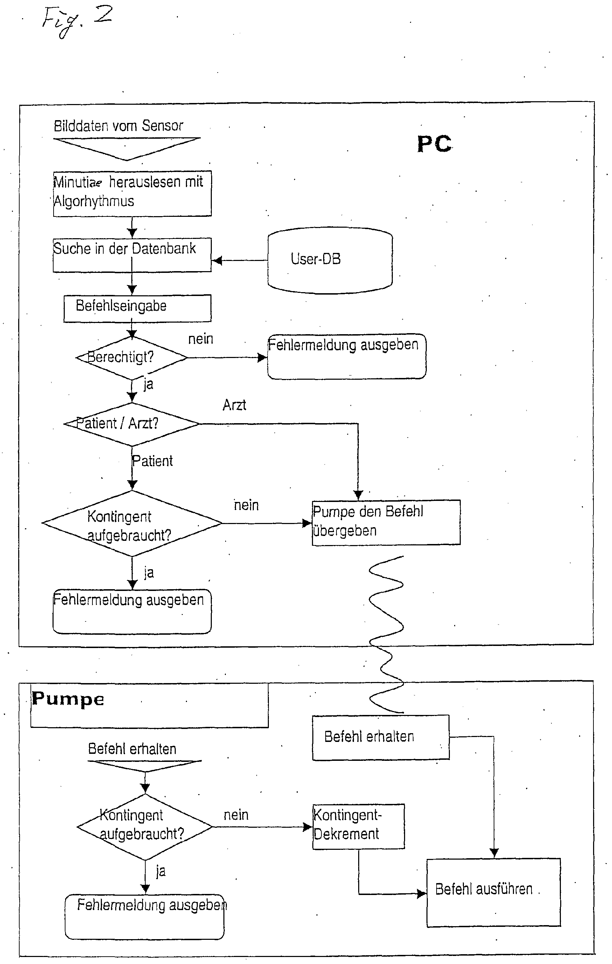

Fig. 2 zeigt eine erste Ausführungsform eines erfindungsgemäßen Ablaufdiagramms zur Überprüfung der Berechtigung eines Bedieners eines medizinischen Geräts, wie z.B. einer Pumpe für medizinische Substanzen, beispielsweise Insulin. In der gezeigten Ausführungsform wird die Überprüfung der Berechtigung eines Benutzers separat von dem medizinischen Gerät durch eine geeignete Vorrichtung, wie z.B. einen PC mit einem Sensor durchgeführt.

Ein Sensor erfasst Bilddaten eines Benutzers, insbesondere zur Identifikation einer Person geeignete Daten, wie z.B. die Struktur der Netzhaut, einen Fingerabdruck und/oder andere geeignete biometrische Merkmale. Aus den erfassten Bilddaten werden charakteristische Details oder Einzelheiten, sogenannte "Minutiae" herausgelesen, wobei durch einen Algorithmus charakteristische Muster oder Merkmale erkannt werden können. Anschließend wird in einer Datenbank, in welcher vorher korrespondierende Merkmale von berechtigten Personen gespeichert wurden, gesucht, ob die durch den Algorithmus ermittelten Merkmale mit in der Datenbank gespeicherten Merkmalen übereinstimmen. Ist dies der Fall, so wird die das Gerät bedienende Person anhand der- vom Sensor erfassten Bilddaten erkannt und es kann eine Berechtigungsinformation zur Verfügung gestellt werden, welche definiert, ob bestimmte Aktionen von dieser Person durchgeführt werden dürfen oder nicht.

Anhand dieser Berechtigungsinformation kann z.B. ermittelt werden, ob ein eingegebener Befehl ausgeführt werden soll und im Falle fehlender Berechtigung kann eine Fehlermeldung ausgegeben werden. Ist die so identifizierte Person zur Eingabe des entsprechenden Befehls berechtigt, so kann in einem weiteren Schritt optional ermittelt werden, ob es sich bei der identifizierten Person z.B. um einen Arzt oder einen Patienten handelt. So kann beispielsweise im Falle der Identifizierung eines Arztes, welcher zur Eingabe aller Befehle berechtigt ist, der Befehl unmittelbar an die mit dem PC gekoppelte Pumpe übergeben werden.

Wird festgestellt, dass der PC von einem Patienten bedient wurde, so kann in einem weiteren Schritt überprüft werden, ob ein für den Patienten vorgesehenes Kontingent bereits aufgebraucht ist oder nicht. Falls dies so ist, kann eine Fehlermeldung ausgegeben werden, wodurch z.B. verhindert werden kann, dass sich Patienten zu viel Schmerzmittel verabreichen. Falls das Kontingent noch nicht aufgebraucht wurde, ist der Patient zur Eingabe des entsprechenden Befehls, z.B. zur Auslösung der Abgabe einer bestimmten Dosis einer Substanz durch die Pumpe, berechtigt und der Befehl kann an die Pumpe übermittelt werden.

In der Pumpe selbst kann der erhaltene Befehl entweder unmittelbar ausgeführt werden oder es kann eine weitere Überprüfung des extern eingegebenen oder eines direkt in die Pumpe eingegebenen Befehls in der Pumpe durchgeführt werden. So ist es beispielsweise möglich, dass auch in der Pumpe nochmals überprüft wird, ob ein Kontingent eines Patienten bereits aufgebraucht ist oder nicht, wobei in Abhängigkeit von dieser Überprüfung entweder eine Fehlermeldung ausgegeben wird oder der Befehl ausgeführt wird und das für den Patienten zur Verfügung stehende Kontingent um die abgegebene Dosis verringert wird.

Es ist auch möglich, dass von der Pumpe eine Information an den PC z.B. bezüglich der abgegebenen Menge der Substanz übermittelt wird, um z.B. auch im PC Informationen über direkt an der Pumpe ausgelöste Infusionen zu haben.

So kann z.B. bei Schmerzmittelpatienten effektiv verhindert werden, dass ein Benutzer sich auf irgendeine Art selbst eine Überdosis eines Schmerzmittels verabreicht oder die versehentliche Bedienung eines medizinischen Geräts z.B. durch spielende Kinder kann ausgeschlossen werden.

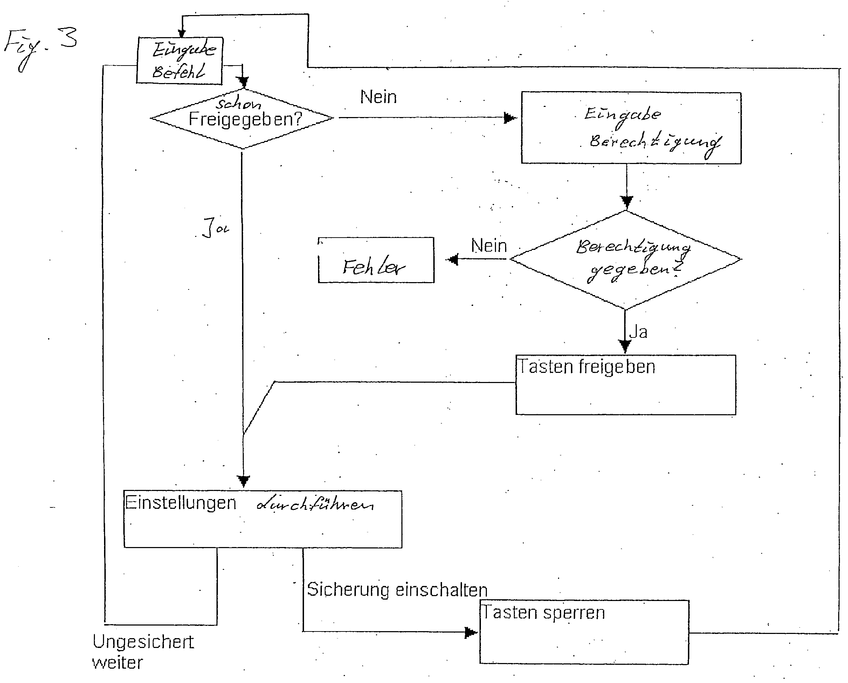

Fig. 3 zeigt ein Ablaufdiagramm einer zweiten Ausführungsform eines Verfahrens, wobei eine elektronische Identifizierungsvorrichtung oder ein sogenannter "Dongle" eingesetzt wird, um die Berechtigung einer Person zur Bedienung eines medizinischen Geräts, wie z.B. einer Pumpe, zu überprüfen.

Ein Benutzer gibt einen Befehl in das medizinische Gerät oder eine mit dem Gerät gekoppelte oder koppelbare Vorrichtung ein und wird z.B. über einen Fingerabdruck, einen Freigabe-Code und/oder einen Dongle erkannt. Vorteilhaft ist z.B. nur ein Dongle pro Pumpe vorgesehen, so dass die Pumpe nur die mit der korrespondierenden Seriennummer versehenen von dem Dongle ausgesendeten Befehle akzeptiert. Nach erfolgter Identifikation und Berechtigungsüberprüfung des Benutzers kann die Pumpe normal bedient werden ohne dass weitere Berechtigungsüberprüfungen erforderlich sind. Optional kann eine zeitliche Begrenzung der Berechtigung zur Eingabe von Befehlen vorgesehen sein, so dass eine versehentliche Freischaltung einer Pumpe nach der vorgegebenen Zeit automatisch beendet wird. Weiterhin ist es möglich, die Freigabe der Pumpe zur Eingabe von Befehlen aktiv zu beenden, so dass nach der Bedienung der Pumpe durch eine berechtigte Person weitere Eingaben an der Pumpe verhindert werden können.

Somit kann die Pumpe z.B. vor einer Betätigung durch Kinder gesichert werden. Weiterhin ist es auch möglich, dass eine Pumpe von einem Benutzer z.B. zur Verwendung in einem Büro freigeschaltet wird und bei der Verwendung zuhause die Sicherung z.B. zum Schutz vor einer unbeabsichtigten Aktivierung durch Kinder eingeschaltet wird.

Es ist auch möglich, dass eine die Durchführung aller Aktionen ermöglichende Berechtigung z.B. in Form eines sogenannten "Master-Keys" vorgesehen ist, Welcher z.B. Ärzten oder anderen Personen zur Verfügung gestellt wird, so dass beispielsweise jede beliebige Pumpe von einem Arzt freigeschaltet oder eingestellt werden kann.

## Patentansprüche

1. Medizinische Vorrichtung (i) umfassend eine Insulinpumpe,
mit einem Bedienelement (2) zum Auslösen einer Aktion der medizinischen Vorrichtung (1), wobei die Aktion die Abgabe eines Bolus durch Infusion ist und wobei die Insulinpumpe in einer Hosentasche oder unter den Kleidern eines Anwenders mitgeführt werden kann,
**dadurch gekennzeichnet, dass** die medizinische Vorrichtung (1) ein Sicherheitssystem (3) umfasst, wobei das Sicherheitssystem (3) einen Fingerprintsensor aufweist und feststellen kann, ob eine Person das Bedienelement (2) betätigt, und eine Auslösung der Aktion andernfalls verhindert.

2. Vorrichtung nach einem der vorhergehenden Ansprüche mit einer Datenbank, in welcher Informationen zur Identifikation mindestens einer Person und/oder zur Berechtigungsstufe einer identifizierten Person gespeichert sind.

3. Vorrichtung nach einem der vorhergehenden Ansprüche mit einer Ausrichtungsvorrichtung, welche das Erfassen eines biometrischen Merkmals in einem vorgegebenen Lageverhältnis einer Fingerkuppe zum Fingerprintsensor sicherstellt.

4. Vorrichtung nach einem der vorhergehenden Ansprüche, wobei das Sicherheitssystem (3) in einer von der Insulinpumpe separaten Vorrichtung angeordnet sind, welche über Funk- oder Infrarotsignale mit der Insulinpumpe koppelbar ist.

5. Vorrichtung nach einem der vorhergehenden Ansprüche, wobei das Sicherheitssystem (3) mindestens eine Person identifizieren kann und feststellen kann, ob die identifizierte Person zur Auslösung der Aktion berechtigt ist.

6. Vorrichtung nach einem der vorhergehenden Ansprüche mit einer optischen und/oder akustischen Ausgabevorrichtung zum Ausgeben eines Betriebszustandes der Vorrichtung.

## Claims

1. Medical apparatus (1) comprising an insulin pump,
with an operating element (2) to trigger an action of the medical apparatus (1), wherein the action is the delivery of a bolus by infusion, and wherein the insulin pump can be carried in a trouser pocket or under the clothing of a user,
**characterized in that** the medical apparatus (1) comprises a security system (3), wherein the security system (3) includes a fingerprint sensor and can determine whether a person is activating the operating element (2) and otherwise prevents the action from being triggered.

2. Apparatus according to any one of the preceding claims, with a database in which information for identifying at least one individual and/or about the authorisation level of an identified individual is stored.

3. Apparatus according to any one of the preceding claims, with an alignment device which ensures the capture of a biometric characteristic at a predetermined positional relationship of a fingertip to the fingerprint sensor.

4. Apparatus according to any one of the preceding claims, wherein the security system (3) is arranged in a separate device from the insulin pump, which can be coupled to the insulin pump via wireless or infrared signals.

5. Apparatus according to any one of the preceding claims, wherein the security system (3) can identify at least one individual and can determine whether the identified individual is authorised to trigger the action.

6. Apparatus according to any one of the preceding claims, with an optical and/or acoustic emission device for emitting an operating state of the apparatus.

## Revendications

1. Dispositif médical (1) comprenant une pompe à insuline,
comportant un élément de manoeuvre (2) pour déclencher une action du dispositif médical (1), l'action étant la délivrance d'un bol par perfusion et la pompe à insuline pouvant être emportée dans une poche de pantalon ou sous les vêtements d'un utilisateur,
**caractérisé en ce que** le dispositif médical (1) comprend un système de sécurité (3), le système de sécurité (3) présentant un capteur d'empreintes digitales et pouvant constater si une personne confirme l'élément de manoeuvre (2) et empêche autrement un déclenchement de l'action.

2. Dispositif selon une des revendications précédentes, comportant une banque de données dans laquelle des informations concernant l'identification d'au moins une personne et/ou le niveau d'autorisation d'une personne identifiée sont enregistrées.

3. Dispositif selon une des revendications précédentes, comportant un dispositif d'orientation qui garantit la détection d'une caractéristique biométrique dans un rapport de position prédéfini d'une phalange par rapport au capteur d'empreintes digitales.

4. Dispositif selon une des revendications précédentes, dans lequel le système de sécurité (3) est disposé dans un dispositif séparé de la pompe à insuline et qui peut être couplé par des signaux radio ou infrarouges à la pompe à insuline.

5. Dispositif selon une des revendications précédentes, dans lequel le système de sécurité (3) peut identifier au moins une personne et peut constater si la personne identifiée est autorisée à déclencher l'action.

6. Dispositif selon une des revendications précédentes, comportant un dispositif d'édition optique et/ou acoustique pour éditer un état de fonctionnement du dispositif.
